# EUROPÄISCHE PATENTSCHRIFT

(11) **EP 1 651 156 B1**
(45) Veröffentlichungstag und Bekanntmachung des Hinweises auf die Patenterteilung: **16.01.2008**
(21) Anmeldenummer: 04763490.2
(22) Anmeldetag: 26.07.2004
(51) Int. Cl.: A61F 13/56, A61F 13/64

(54) **WEGWERFWINDEL MIT HÜFTGÜRTEL**
DISPOSABLE DIAPER WITH A GIRDLE
COUCHE JETABLE A CEINTURE

(30) Priorität: 06.08.2003 DE 10337537
(43) Veröffentlichungstag der Anmeldung: 03.05.2006
(73) Patentinhaber: Paul Hartmann Aktiengesellschaft, 89522 Heidenheim (DE)
(72) Erfinder: RÖHRL, Wolfgang, 89542 Herbrechtingen (DE); STUPPERICH, Hans-Peter, 89522 Heidenheim (DE)
(74) Vertreter: Friz, Oliver
(86) Internationale Anmeldenummer: PCT/EP2004/008335
(87) Internationale Veröffentlichungsnummer: WO 2005/016210

(56) Entgegenhaltungen:
- WO-A-91/08725
- WO-A-96/19169
- WO-A-03/007865
- FR-A- 2 586 558
- GB-A- 1 520 740
- US-A- 4 850 992
- US-A- 5 069 678
- US-A- 5 370 634
- US-A1- 2001 034 511
- US-A1- 2002 095 132

## Beschreibung

Die Erfindung betrifft eine Wegwerfwindel, insbesondere zur Inkontinentenversorgung, mit einem eine in Umfangsrichtung geschlossene Hüftöffnung der Windel bildenden Hüftgürtel, der an wenigstens einer Stelle öffenbar und schließbar ist, und mit einem einen Vorderbereich, einen Rückenbereich und einen dazwischenliegenden Schrittbereich aufweisenden Windelhauptteil, der eine körperabgewandte flüssigkeitsundurchlässige Lage (Backsheet), eine körperzugewandte flüssigkeitsdurchlässige Lage (Topsheet) und einen Absorptionskörper für Körperflüssigkeiten aufweist, wobei der Hüftgürtel unlösbar an den Rückenbereich des Windelhauptteils angefügt ist und der Windelhauptteil mit dem Längsende seines Vorderbereichs über erste Verschlussmittel lösbar am Hüftgürtel festlegbar ist, derart, dass ein Benutzer bei angelegtem Hüftgürtel den Windelhauptteil zwischen den Beinen hervorholen und das noch freie Längsende des Windelhauptteils am Hüftgürtel lösbar festlegen kann.

Derartige Wegwerfwindeln sind vielfach bekannt geworden. Sie bieten den Vorteil, dass ein Benutzer beim Anlegen der Windel zunächst den Hüftgürtel um die Hüften herumlegt, und üblicherweise im Bauchbereich schließen kann. Währenddessen hängt der üblicherweise mit seinem Rückenbereich am Gürtel befestigte Windelhauptteil der Wegwerfwindel lose nach unten. Der Benutzer ergreift dann nach dem Schließen des Hüftgürtels das freihängende Ende des Windelhauptteils und führt den Windelhauptteil von hinten zwischen den Beinen hervor, um den Windelhauptteil mit seinem freien Längsende am Hüftgürtel innen oder außen lösbar festzulegen. Hierfür sind die ersten Verschlussmittel vorgesehen. Es versteht sich, dass das Anlegen der Wegwerfwindel auch so ausgeführt werden kann, dass nach dem Anlegen und Schließen des Hüftgürtels der frei nach unten hängende Windelhauptteil von vorn nach hinten zwischen den Beinen eines Benutzers hindurchgeführt und solchenfalls mit seinem Rückenbereich am Hüftgürtel lösbar befestigt werden kann. Es sind aber auch Wegwerfwindeln bekannt geworden, bei denen der Windelhauptteil ganz vom Hüftgürtel lösbar ist, so dass insbesondere bei stark pflegebedürftigen und immobilen Benutzern eine weitgehende Flexibilität bei der Handhabung der Wegwerfwindel gewährleistet ist.

Aus WO-A-03/007865 ist ein absorbierender Artikel in Form eines Unterbekleidungsstücks mit einem in Umfangsrichtung durchgehenden oder auf sich selbst schließbaren Hüftbereich und einem Schrittbereich und mit den weiteren Oberbegriffsmerkmalen des Anspruchs 1 bekannt. Ferner sind zwischen dem Schrittbereich und dem Hüftbereich kurze zweite Verschlussmittel vorgesehen, welche im geschlossenen Zustand die Beinöffnungen fixieren.

Hiervon ausgehend liegt der vorliegenden Erfindung die Aufgabe zugrunde, die Verwendbarkeit einer gattungsgemäßen Wegwerfwindel zu erweitern.

Diese Aufgabe wird erfindungsgemäß durch eine Wegwerfwindel mit den Merkmalen des Anspruchs 1 gelöst.

Mit der Erfindung wird also vorgeschlagen, eine Wegwerfwindel, insbesondere zur Versorgung inkontinenter Erwachsener und zur Benutzung durch inkontinente Erwachsene, so auszubilden, dass der Benutzer oder Pflegepersonal die Wegwerfwindel zu Inspektionszwecken wie vorausgehend beschrieben öffnen kann. Dabei bleiben die zweiten Verschlussmittel vorzugsweise geschlossen. Lediglich die ersten Verschlussmittel zwischen Windelhauptteil und Hüftgürtel werden geöffnet und der Windelhauptteil wird dann vom Hüftgürtel weg geklappt oder gefaltet, um eine Einsichtnahme in das Innere der Windel zu gestatten. Es kann somit geprüft werden, ob die Windel schon verunreinigt, also gebraucht ist, oder noch für die Aufnahme von flüssigen oder festen Körperausscheidungen weiter benutzt werden kann. Es erweist sich als ganz besonders vorteilhaft, dass die Windel hierzu nur durch Lösen der ersten Verschlussmittel teilweise geöffnet werden muss, ansonsten aber im bestimmungsgemäß angelegten Zustand am Körper verbleibt. Durch die stützende Funktion der zweiten Verschlussmittel verbleibt die Wegwerfwindel in der beim Anlegen gewählten optimalen Positionierung. Eine Inspektion der Windel ist daher sehr rasch und für den Benutzer wenig störend ausführbar.

Die erfindungsgemäße Wegwerfwindel bietet aber auch einen weiteren Vorteil, in dem sie nicht nur als echter Inkontinenzartikel verwendet werden kann, sondern bei Bedarf zur bevorzugten Verwendung bei männlichen Benutzern eher wie ein Unterbekleidungsstück gehandhabt wird, indem die Wegwerfwindel im Vorderbereich teilweise geöffnet wird, ansonsten aber mit geschlossenem Hüftgürtel weiter angelegt bleibt, während der Benutzer Wasser lassen kann. Solchenfalls klappt oder faltet der Benutzer den Windelhauptteil zumindest soweit vom Hüftgürtel weg, dass er im ansonsten angelegten Zustand der Windel bei geschlossen gehaltenen zweiten Verschlussmitteln Wasser lassen kann, ohne die Windel zu beaufschlagen. Dieser Vorteil erweist sich als ganz besonders zweckmäßig bei Verwendung der erfindungsgemäßen Wegwerfwindel durch solche Benutzer, bei denen nur zeitweise die Kontrolle über das Wasserlassen beeinträchtigt ist oder die üblicherweise den Drang, Wasser lassen zu müssen, verspüren und eine entsprechende Bequemlichkeit aufsuchen können, sofern diese in unmittelbarer Nähe zur Verfügung steht. Solchenfalls können die Benutzer bei angelegtem Hüftgürtel die ersten Verschlussmittel lösen und so den Vorderbereich des Windelhauptteils vom Hüftgürtel weg nach unten klappen oder falten, so dass insbesondere männliche Benutzer dann ihr Glied ergreifen und in üblicher Weise zum Wasserlassen nach außen zu führen vermögen, so dass die Wegwerfwindel nicht mit Flüssigkeit beaufschlagt wird.

Die Möglichkeit einer solchen Verwendung einer Wegwerfwindel ist in ihrer Auswirkung auf die Psyche des Benutzers nicht hoch genug einzuschätzen. Der Benutzer verliert dadurch das Gefühl, dass er auf typische Inkontinenzartikel angewiesen sein soll, auch wenn dies teilweise oder in weitgehenderem Maße tatsächlich der Fall sein sollte. Insbesondere im Kontakt zwischen Arzt oder Pflegepersonal und Patient erleichtert die erfindungsgemäße Ausbildung der Wegwerfwindel die Kommunikation und die Akzeptanz der auferlegten oder angeratenen Verwendung von Inkontinenzartikeln der hier in Rede stehenden Art.

Wenn vorausgehend von langgestreckt ausgebildeten zweiten Verschlussmitteln die Rede ist, so wird hierunter eine vorzugsweise streifen- oder bandförmige Gestalt der zweiten Verschlussmittel verstanden, wobei deren Breite nicht zwangsläufig ausgesprochen schmal zu sein braucht, obschon dies eine größtmögliche Flexibilität bei der Handhabung gewährleistet. Die zweiten Verschlussmittel haben zweckmäßigerweise eine Länge von 10 bis 40 cm und weiter insbesondere von 15 bis 35 cm. Sie können in Einzelfällen, beispielsweise bei Wegwerfwindel für besonders korpulente Personen, aber auch demgegenüber länger ausgebildet sein. Die Breite der zweiten Verschlussmittel quer zu ihrer Längserstreckung liegt zweckmäßigerweise zwischen 0,5 und 8 cm, wobei ein Bereich von 0,5 bis 6 cm, insbesondere bis 5 cm, insbesondere bis 4 cm und vorzugsweise bis 3 cm bevorzugt wird.

Es sind zweckmäßigerweise zwei solche langgestreckte zweiten Verschlussmittel vorgesehen, die sich von einem unteren, also gürtelabgewandten Bereich des Vorderbereichs oder gar vom Schrittbereich des Benutzers in Richtung auf seine Hüften zu den Seiten hin erstrecken. Zwischen diesen beiden zweiten Verschlussmitteln vermag dann der Vorderbereich des Windelhauptteils der Wegwerfwindel, nachdem er vom Hüftgürtel gelöst wurde, nach vorn oder nach unten oder aber nach innen, in jedem Fall aber vom Hüftgürtel weg geklappt oder gefaltet zu werden. Die zweiten Verschlussmittel sind daher im angelegten Zustand der Windel zweckmäßigerweise etwa V-förmig zueinander verlaufend angeordnet. Sie müssen sich aber nicht notwendigerweise im Bereich ihrer Enden schneiden. Der Winkel zu einer auf den Benutzer projizierten Längsmittelachse der Wegwerfwindel dürfte in vorteilhafter Weise zwischen 5 und 60°, insbesondere zwischen 5 und 50° und weiter insbesondere zwischen 10 und 50° betragen. Es wird aber ausdrücklich darauf hingewiesen, dass nicht notwendigerweise zwei voneinander separate langgestreckte Verschlussmittel vorgesehen sein müssen. Beispielsweise wäre es denkbar und für bestimmte Zwecke, Ausbildungen und Anwendungen vorteilhaft, wenn die zweiten Verschlussmittel durch ein einziges V-förmiges oder bogenförmiges Element gebildet sind.

Dadurch dass die zweiten Verschlussmittel elastisch dehnbar sind, brauchen die zweiten Verschlussmittel nicht notwendigerweise weit nach unten, also in Richtung auf den Schrittbereich des Windelhauptteils, erstreckt zu werden, sondern bei geeigneter Konfiguration der Verschlussmittel können diese beim Wegfalten des Vorderbereichs des Windelhauptteils zum Zwecke des Wasserlassens auch gedehnt werden.

Wenn vorausgehend davon die Rede ist, dass die zweiten Verschlussmittel dehnbar sind, so wird hierunter verstanden dass sie gegenüber ihrer Ausgangslänge im unbelasteten Zustand um wenigstens 10%, vorzugsweise um wenigstens 50% durch Einleitung entsprechender Zugkräfte gedehnt werden können. Wenn vorausgehend von einer elastischen Dehnbarkeit die Rede ist, so wird hierunter verstanden, dass ein gedehntes Material bestrebt ist, sich nach Wegnahme der Dehnungskräfte wieder zusammenzuziehen. Man spricht von elastischer Dehnbarkeit, wenn sich ein auf wenigstens 130%, vorzugsweise auf wenigstens 150% seiner Ausgangslänge gedehntes Material wieder derart zusammenzieht, dass die zuvor durch Dehnung herbeigefügte Längung um wenigstens 50% rückgängig gemacht wird, und zwar vorzugsweise sofort, längstens innerhalb von drei Minuten. Dies bedeutet in Zahlen ausgedrückt, dass ein Material von 10 cm Ausgangslänge nach einer Dehnung auf 15 cm Ausgangslänge sich wieder auf eine Länge von höchstens 12,5 cm zusammenzieht.

Die Funktion der zweiten Verschlussmittel liegt insbesondere darin, dass sie auch bei geöffneten ersten Verschlussmitteln die Windel am Körper des Benutzers in ihrer bestimmungsgemäßen Position halten, und zwar vorzugsweise auch dann, wenn die Wegwerfwindel bzw. ihr Absorptionskörper beträchtliche Mengen an Flüssigkeit hält, welche den Windelhauptteil der Windel nach unten zu ziehen versuchen. Ungeachtet dessen unterstützen die langgestreckten zweiten Verschlussmittel eine passgenaue Anordnung der Windel am Körper des Benutzers. Die zweiten Verschlussmittel können insbesondere so angelegt werden, dass sie eine Vorspannung auf den angrenzenden Windelhauptteil der Wegwerfwindel ausüben und diesen in enger Anlage an den Körper zwingen, ohne dass es hierbei jedoch zu unangenehmem Einschneiden kommen soll. Gerade bei mobilen Benutzern mit ansonsten geringer Beschränkung körperlicher und geistiger Funktionen können die zweiten Verschlussmittel auch dazu dienen, die Wegwerfwindel in einem wenig auftragenden Zustand am Körper zu halten, was wiederum in psychologischer Hinsicht wertvoll sein kann.

Wie vorausgehend erwähnt, kann es sich bei den langgestreckten zweiten Verschlussmitteln um insbesondere zwei streifen- oder bandförmige Verschlussmittel mit zwei gegenüberliegenden freien Enden handeln. Nach einer Ausführungsform der Erfindung sind die Verschlussmittel mit jeweils einem freien Ende beim bestimmungsgemäßen Gebrauch unlösbar mit dem Hüftgürtel verbunden. Ihr gegenüberliegendes anderes Ende ist frei und kann beim Anlegen der Wegwerfwindel an den Körper an der Außenseite des Windelhauptteils, vorzugsweise im unteren Vorderbereich oder Schrittbereich des Windelhauptteils, lösbar festgelegt werden. Nach einer anderen Ausführungsform können die langgestreckten zweiten Verschlussmittel mit dem Windelhauptteil, insbesondere mit dem Vorderbereich oder Schrittbereich des Windelhauptteils beim bestimmungsgemäßen Gebrauch unlösbar verbunden sein, wobei dann die lösbare Halteverbindung im Bereich ihres anderen Endes mit dem Hüftgürtel hergestellt bzw. gelöst werden kann.

Auch eine noch weitere Ausführungsform ist denkbar, wonach die zweiten Verschlussmittel mit ihren beiden Enden, insbesondere unlösbar am Hüftgürtel und am Windelhauptteil festgelegt sind und entlang ihrer Erstreckung eine lösbar schließbare Verbindung aufweisen. Diese Verbindung kann dann beispielsweise eine voreingestellte Schnalle oder eine in Bezug auf ihre Anordnung am langgestreckten Verschlussmittel verstellbare Schnalle oder klebende oder mechanisch wirkende Verschlusselemente aufweisen.

An dieser Stelle sei erwähnt, dass die ersten, aber auch die zweiten Verschlussmittel für sich genommen bekannte mechanisch wirkende Verschlusselemente, insbesondere ein Haken bzw. Schlaufen aufweisendes Material, oder aber klebend wirkende Bereiche aufweisen können. Sowohl klebende als auch mechanisch wirkende Verschlusselemente sind hinreichend bekannt und üblich, so dass eine detaillierte Beschreibung entfallen kann. Es sei nur noch erwähnt, dass die ersten Verschlussmittel, aber auch die zweiten Verschlussmittel und die mit ihnen zusammenwirkenden Gegenverschlusselemente am Windelhauptteil oder am Hüftgürtel entweder so angeordnet sein können, dass es dem Benutzer überlassen bleibt, wo er die Halteverbindung ausbildet oder mit welcher Zugspannung er eine Halteverbindung ausbildet. Es besteht indessen auch die Möglichkeit, die ersten und/oder die zweiten Verschlussmittel über vorpositionierte Gegenverschlusselemente oder Gegenverschlussbereiche am Windelhauptteil oder Hüftgürtel zu schließen. Auf diese Weise wird vom Benutzer eine weitestgehend korrekte Positionierung der Verschlussmittel erzwungen. Solchenfalls erweist sich eine elastisch nachgiebige Ausbildung der eingesetzten Materialien, insbesondere der Verschlussmittel, als vorteilhaft, um verschiedene Körpergrößen und Körpergestalten der Benutzer akkomodieren zu können.

Nach einer besonders bevorzugten Ausführungsform der vorliegenden Erfindung sind die zweiten Verschlussmittel bei der Abgabe an den Verbraucher, insbesondere im verpackten Zustand vor der Ingebrauchnahme jeweils für sich insbesondere leporelloförmig aufeinandergefaltet.

Nach einer weiteren vorteilhaften Ausführungsform der Erfindung sind die zweiten Verschlussmittel bei der Abgabe an den Verbraucher, insbesondere im verpackten Zustand vor der Ingebrauchnahme, entlang dem Hüftgürtel angeordnet oder erstreckt. Sie können dabei mit einem Ende mit dem Hüftgürtel verbunden sein, wobei hierunter sowohl ein Anfügen der zweiten Verschlussmittel an den Hüftgürtel oder eine einstückige Ausbildung von Hüftgürtel.und Verschlussmittel verstanden wird. Beispielsweise könnte der Hüftgürtel in Umfangsrichtung einen Trennschnitt aufweisen, wodurch das zweite Verschlussmittel als vormals langgestreckter bandförmiger Abschnitt des Hüftgürtels herausgebildet wird. In besonders vorteilhafter Weiterbildung dieses Erfindungsgedankens könnte der das zweite Verschlussmittel bildende Abschnitt des Hüftgürtels über Sollbruchstellen noch am Hüftgürtel angeordnet sein.

Nach einer anderen Ausführungsform der Erfindung könnten die zweiten Verschlussmittel bei der Abgabe an den Verbraucher, insbesondere im verpackten Zustand vor der Ingebrauchnahme, an der flüssigkeitsundurchlässigen Lage angeordnet sein. Auch dort könnten sie in an sich beliebiger Weise lösbar gehalten sein.

In noch weiterer Ausbildung der Erfindung wird vorgeschlagen, dass die zweiten Verschlussmittel bei der Abgabe an den Verbraucher, insbesondere im verpackten Zustand vor der Ingebrauchnahme, dem Hüftgürtel oder dem Windelhauptteil derart beigeordnet sind, dass sie in dieser Konfiguration auch ungebraucht belassen werden können, ohne störend zu wirken. Sie könnten auch einer Verpackung losgelöst vom Hüftgürtel und vom Windelhauptteil beigegeben sein. Soll beispielsweise eine erfindungsgemäß ausgebildete Wegwerfwindel ausschließlich als reiner Inkontinenzartikel Verwendung finden oder wird in einer bestimmten Pflegesituation Wert auf ein rasches Anlegen oder Abnehmen des Hygieneartikels, insbesondere durch den Benutzer selbst, gelegt werden, so erweist sich die beschriebene Anordnung oder Beiordnung der zweiten Verschlussmittel als vorteilhaft.

In Weiterbildung dieses Erfindungsgedankens wird vorgeschlagen, die Verschlussmittel derart am Hüftgürtel oder Windelhauptteil anzuordnen, dass sie bei der Abgabe an den Verbraucher, insbesondere im verpackten Zustand vor der Ingebrauchnahme, kaschiert sind. Dies kann etwa dadurch geschehen, dass sie von weiteren Deckelementen oder Deckschichten überfangen sind. Es wäre auch denkbar, an entsprechender Stelle des Hüftgürtels oder des Windelhauptteils taschenförmige Aufnahmemittel vorzusehen, oder ein zweites insbesondere leporelloförmig auch sich selbst gefaltetes Verschlussmittel durch einen lösbaren Tapestreifen in dieser Konfiguration zu halten oder zu verdecken.

Nach einem weiteren für sich genommen selbständigen Erfindungsgedanken, der bei Wegwerfwindeln der gattungsgemäßen Art Anwendung finden kann, sind die in Querrichtung vom Windelhauptkörper sich wegerstreckenden Abschnitte des Hüftgürtels insbesondere leporelloförmig (Z-förmig) auf sich selbst und/oder in Richtung auf eine Längsmittelachse der Wegwerfwindel gefaltet. In Weiterbildung dieses selbständigen Erfindungsgedankens sind die sich beidseits vom Windelhauptkörper wegerstreckenden Abschnitte des Hüftgürtels bei der Abgabe an den Verbraucher, insbesondere im verpackten Zustand vor der Ingebrauchnahme, einander überlappend in Richtung auf die Längsmittelachse der Windel gefaltet. Dabei erstrecken sich ihre freien Enden vorzugsweise nach Außen, also von der Windellängsachse weg, damit sie durch einen Benutzer der Wegwerfwindel gut greifbar sind und der Hüftgürtel infolge des Ergreifens entfaltbar ist.

In weiterer Ausbildung dieses Erfindungsgedankens ist der Hüftgürtel von einem einzigen sich in Umfangsrichtung erstreckenden Materialabschnitt gebildet. In noch weiterer Ausbildung des Erfindungsgedankens ist der insbesondere einstückig ausgebildete Hüftgürtel derart nach Innen auf den Windelhauptteil gefaltet, dass sich eine Faltung des einen Gürtelabschnitts in eine Faltung des anderen Gürtelabschnitts hineinerstreckt.

Weitere Merkmale, Einzelheiten und Vorteile der Erfindung ergeben sich aus den beigefügten Patentansprüchen und der zeichnerischen Darstellung und nachfolgenden Beschreibung bevorzugter Ausführungsformen der erfindungsgemäßen Wegwerfwindel. In der Zeichnung zeigt:
- Figur 1: die erfindungsgemäße Wegwerfwindel im angelegten Zustand;
- Figur 2: eine Draufsicht auf die Wegwerfwindel nach Figur 1 im flachgelegten Zustand;
- Figur 3: eine Figur 2 entsprechende Draufsicht auf eine weitere Ausführungsform der erfindungsgemäßen Wegwerfwindel;
- Figur 4: eine schematische Ansicht einer weiteren Ausführungsform einer erfindungsgemäßen Wegwerfwindel im angelegten Zustand;
- Figur 5: eine Draufsicht auf die Wegwerfwindel nach Figur 4 im flachgelegten Zustand;
- Figur 5b: eine Figur 5 entsprechende Draufsicht auf eine Wegwerfwindel mit beigeordneten zweiten Verschlussmitteln;
- Figur 6: eine schematische Darstellung der Faltung des Hüftgurts;
- Figur 7: eine schematische Ansicht einer weiteren erfindungsgemäßen Wegwerfwindel im angelegten Zustand und
- Figur 8: ein schematische Darstellung einer weiteren Ausführungsform einer erfindungsgemäßen Wegwerfwindel im angelegten Zustand.

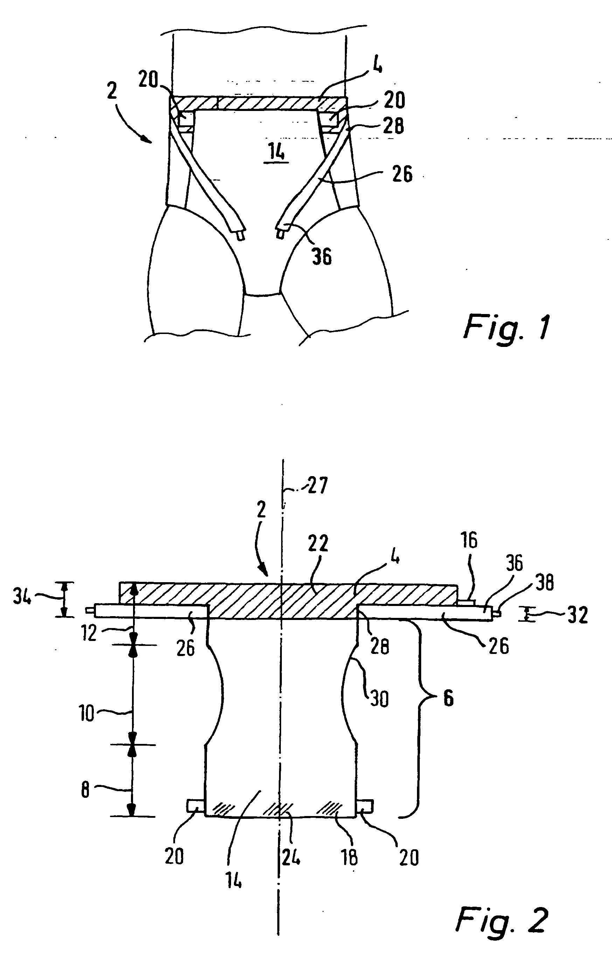

Die Figuren 1 und 2 zeigen eine erste Ausführungsform einer erfindungsgemäßen Wegwerfwindel 2 im angelegten und im flachgelegten Zustand. Die Wegwerfwindel umfasst einen Hüftgürtel 4 und einen daran lösbar oder unlösbar angefügten Windelhauptteil 6, der einen Vorderbereich 8, einen Schrittbereich 10 und einen Rückenbereich 12 umfasst und mit seinem Rückenbereich 12 den Hüftgürtel 4 überlappt und dort vorzugsweise unlösbar mit dem Hüftgürtel 4 verbunden ist. Figur 2 zeigt eine Draufsicht auf eine körperabgewandte flüssigkeitsundurchlässige Lage 14, also auf das Backsheet der Wegwerfwindel. Der Windelhauptteil 6 umfasst neben dem Backsheet einen nicht dargestellten Absorptionskörper und eine ebenfalls nicht dargestellte körperzugewandte flüssigkeitsdurchlässige Lage (Topsheet), die entweder von einer dem Absorptionskörper zuzurechnenden Schicht oder von einer separaten Topsheet-Lage gebildet sein kann.

Der Hüftgürtel 4 umfasst eine solche Länge in Querrichtung der Wegwerfwindel, dass er, wie in Figur 1 schematisch dargestellt, um den gesamten Hüftumfang eines Benutzers herumgelegt und auf sich selbst geschlossen werden kann, so dass er eine in Umfangsrichtung geschlossene Hüftöffnung bildet. Hierfür umfasst der Hüftgürtel Verschlusslaschen 16 oder sonstige an sich beliebige Verschlusselemente, die klebend oder mechanisch wirkend ausgebildet sein können. Nach einer bevorzugten Ausführungsform sind an sich bekannte mechanisch wirkende Verschlusselemente in Form von Haken-/Schlaufenmaterialien vorgesehen. In bevorzugter Ausführung des Hüftgürtels umfasst dieser eine textile oder textilartige Außen- und/oder Innenseite, insbesondere ein Vlies (nonwoven), welche ein Schlaufenmaterial bildet. Diese Außen- und/oder Innenseite kann als Auftreffbereich für eine hakenbildende Komponente an einem freien Ende des Hüftgürtels 4 dienen.

Am freien Längsende 18 des Vorderbereichs 8 des Windelhauptteils 6, und zwar beidseitig links und rechts, sind erste Verschlussmittel 20 in Form von Verschlusslaschen vorgesehen. Diese Verschlusslaschen tragen wiederum an sich beliebige Verschlusselemente. Bevorzugt werden mechanisch wirkende Verschlusselemente, insbesondere in Form einer hakenbildenden Komponente eines Haken-/Schlaufenmaterials, wobei solchenfalls die Außenseite 22 des Hüftgürtels 4 zumindest in einem Auftreffbereich als schlaufenbildende Komponente ausgebildet ist. Es wird aber ausdrücklich darauf hingewiesen, dass die ersten Verschlussmittel 20 nicht als vorstehende Verschlusslaschen ausgebildet sein müssen, sondern, dass es auch denkbar und vorteilhaft sein kann, wenn das Längsende 18 des Vorderbereichs 8 in schraffiert angedeuteten Bereichen 24 oder durchgehend auf der Innen- oder Außenseite mit Verschlusselementen versehen ist, so dass der Windelhauptteil innen oder außen am Hüftgürtel 4 lösbar festgelegt werden kann.

Schließlich zeigen die Figuren zweite Verschlussmittel 26, die langgestreckt und streifen- oder bandförmig ausgebildet sind und sich in der Darstellung nach Figur 2 wie der Hüftgürtel 4 in Umfangsrichtung, also quer zur Längsmittelachse 27 der Wegwerfwindel 2 erstrecken. Im dargestellten Fall sind die zweiten langgestreckten Verschlussmittel 26 mit einem Ende 28 ungefähr im Bereich des Seitenrands 30 des Windelhauptteils 6 an den Hüftgürtel 4 lösbar oder vorzugsweise unlösbar angefügt. Ihre Breite 32 ist im dargestellten Fall geringer als die Breite 34 des Hüftgürtels 4. Sie erstrecken sich im dargestellten Fall in Umfangsrichtung über die freien Enden des Hüftgürtels 4 hinaus. Denkbar wäre aber auch, dass die zweiten Verschlussmittel 26 in Umfangsrichtung vor den freien Enden des Hüftgürtels enden. An ihrem dem Ende 28 gegenüberliegenden Ende 36 tragen die zweiten Verschlussmittel 26 an sich beliebige mechanisch oder klebend ausgebildete Verschlusselemente 38, die im dargestellten Fall in Form von vorstehenden Laschen ausgebildet sind.

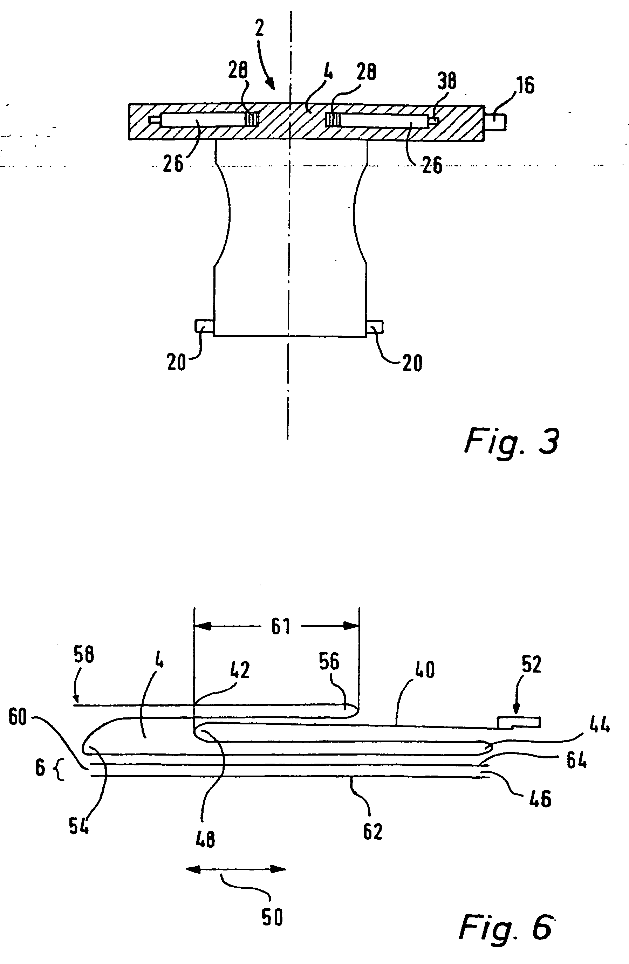

Figur 3 zeigt eine der Figur 2 entsprechende Draufsicht auf eine weitere Ausführungsform einer erfindungsgemäßen Wegwerfwindel. Bei dieser Wegwerfwindel sind die zweiten Verschlussmittel 26 mittig auf dem Hüftgürtel 4 positioniert. Ihre befestigten Enden 28 liegen innerhalb des Hauptteils, und ihre freien Enden 36 sind über ansich beliebige Verschlusselemente 38 am Hüftgürtel 4 lösbar festgelegt.

Figur 1 zeigt die erfindungsgemäße Wegwerfwindel 2 im angelegten Zustand. Üblicherweise wird die Windel so angelegt, dass ein Benutzer zunächst den Hüftgürtel 4 um seine Hüften herum legt, so dass er einen geschlossenen Ring bildet, und den Hüftgürtel im Bauchbereich mittels der dort vorgesehenen Verschlusselemente 16 schließt. Sodann holt der Benutzer den über sein Gesäß frei nach unten hängenden Windelhauptteil 6 zwischen den Beinen hervor und positioniert das freie Längsende 18 des Vorderbereichs 8 des Windelhauptteils 6 innen oder außen am Hüftgürtel 4 und schließt so die Wegwerfwindel 2 über die beschriebenen ersten Verschlussmittel 20. Man spricht hier von einer Primärschließfunktion der Wegwerfwindel. In dieser Funktion ist die Wegwerfwindel 2 insbesondere als Inkontinenzartikel benutzbar. Es wäre denkbar, dass die langgestreckten zweiten Verschlussmittel 26 ungenutzt in ihrer aus Figur 2 ersichtlichen Anordnung am Hüftgürtel verbleiben. Figur 1 zeigt hingegen die zweiten Verschlussmittel 26 in einer aktivierten Schließfunktion. Das freie Ende 36 der beiden Verschlussmittel 26 ist in einem unteren Bereich des Vorderbereichs 8 oder im Schrittbereich 10 auf der Außenseite der Wegwerfwindel 2 lösbar festgelegt. Auf diese Weise kann der Benutzer eine beliebige Zugspannung beim Schließen der zweiten Verschlussmittel 26 auswählen oder vorgeben und so eine zusätzliche Stützwirkung für den Windelhauptteil 6 der Wegwerfwindel erzielen. Im dargestellten Fall der Figur 1. erstrecken sich die zweiten Verschlussmittel 26 im angelegten Zustand seitlich von den Hüften des Benutzers in einem Winkel von ca. 30 bis 50° zur Längsmittelachse 27 nach innen. Sie verlaufen also im wesentlichen V-förmig. Dies eröffnet die Möglichkeit, dass der Benutzer die ersten Verschlussmittel 20 öffnen und so den Vorderbereich 8 des Windelhauptteils 6 vom Hüftgürtel 4 lösen und vom Hüftgürtel weg nach innen oder außen klappen oder falten kann. Sofern die zweiten langgestreckten Verschlussmittel 26 elastisch ausgebildet sind, können diese dabei noch gedehnt werden. Diese neue Verwendbarkeit der erfindungsgemäßen Wegwerfwindel ermöglicht es dem Benutzer, Wasser zu lassen, ohne dass dabei die Wegwerfwindel mit Flüssigkeit beaufschlagt wird. Anschließend kann der Benutzer den Windelhauptteil 6 wieder, wie eingangs beschrieben, am Hüftgürtel 4 befestigen.

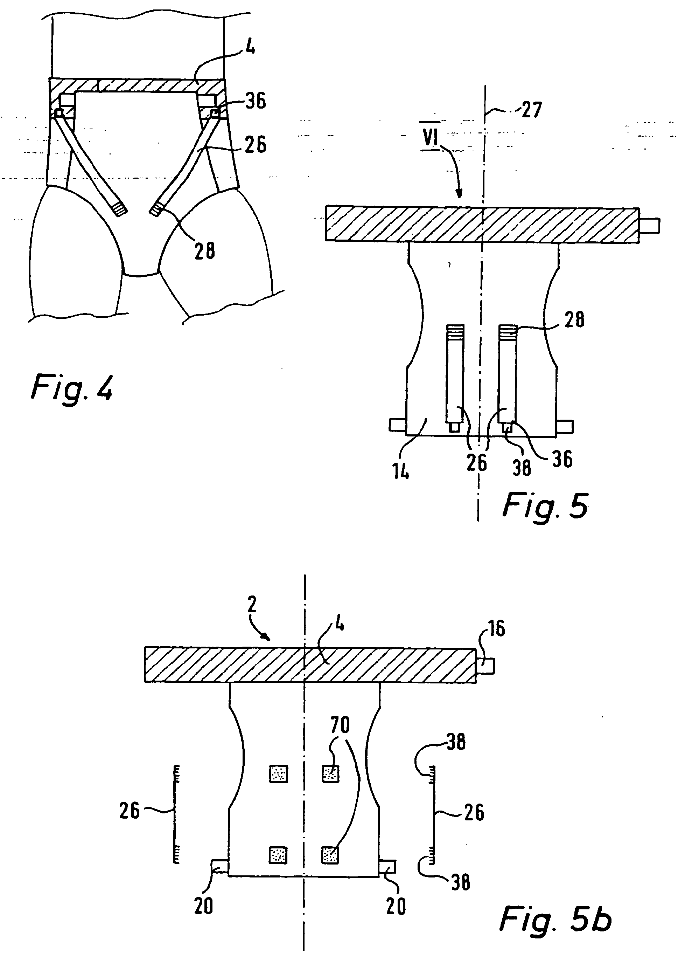

Die Ausführungsformen nach Figuren 4 und 5 zeigen eine Anordnung der zweiten Verschlussmittel 26 an der Außenseite der körperabgewandten flüssigkeitsundurchlässigen Lage 14 (Backsheet). Sie sind in Richtung der Längsmittelachse 27 erstreckt angeordnet. Dabei sind die wiederum langgestreckt ausgebildeten zweiten Verschlussmittel 26 mit einem Ende 28 im Schrittbereich 10 lösbar oder unlösbar festgelegt. Ihr anderes freies Ende 36 ist über wiederum an sich beliebige Verschlusselemente 38 lösbar festgelegt. Es wird aber darauf hingewiesen, dass die zweiten Verschlussmittel 26 der Wegwerfwindel auch beigeordnet sein können. Solchenfalls kann das Backsheet des Windelhauptteils 6 wie beispielsweise in Fig. 5b dargestellt an den mit Bezugsziffer 70 gekennzeichneten Landezonen an sich beliebige, insbesondere mechanisch wirkende oder klebende Gegenverschlusselemente aufweisen, die mit entsprechenden Verschlusselementen 38 der zweiten Verschlussmittel 26 eine Halteverbindung eingehen können. Denkbar und vorteilhaft ist es auch, wenn die gesamte Außenseite des Backsheets so beschaffen ist, dass sie mit den Verschlusselementen 38 der zweiten Verschlussmittel 26 eine Halteverbindung eingehen kann. Solchenfalls wäre es vorteilhaft, die bevorzugten Landezonen 70 für die Verschlusselemente 38 der zweiten Verschlussmittel 26 auf dem Backsheet visuell zu kennzeichnen, um dem Anwender die richtige Positionierung der zweiten Verschlussmittel zu erleichtern.

Figur 4 zeigt die Wegwerfwindel im angelegten Zustand. Man erkennt die festgelegten Enden 28 im Schrittbereich und die lösbar am Hüftgürtel 4 festlegbaren freien Enden 36.

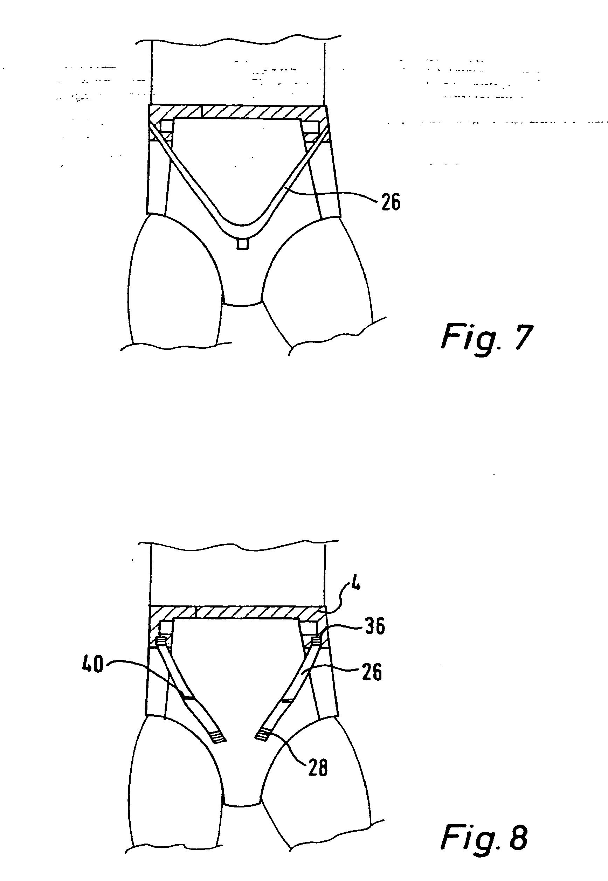

Figur 6 zeigt schematisch eine bevorzugte Faltung des Hüftgürtels 4 auf den Windelhauptteil 6, und zwar gesehen in Richtung des Pfeils VI in der Darstellung nach Figur 5, wobei der Hüftgürtel dort entfaltet dargestellt ist. Man erkennt den aus einem einstückigen Materialabschnitt gebildeten Hüftgürtel 4. Er umfasst einen auf die eine Seite vorstehenden Hüftgürtelabschnitt 40 und einen auf die andere Seite vorstehenden Hüftgürtelabschnitt 42. Zunächst ist der Hüftgürtelabschnitt 40 um eine Achse 44 parallel zur Längsmittelachse im Bereich eines Längsseitenrands 46 des Windelhauptteils 6 nach innen gefaltet. Der Abschnitt 40 ist dann um eine zweite Faltachse 48 wiederum auf sich selbst nach Außen gefaltet. Die Position der zweiten Faltachse 48 in angedeuteter Querrichtung 50 ist dabei in bevorzugter Ausführung dieses ansich selbständigen Erfindungsgedankens so positioniert, dass ein freier Endabschnitt 52 des Hüftgürtels 4 im Bereich des Längsseitenrands 46 des Windelhauptteils für einen Benutzer manuell gut greifbar angeordnet ist. Das freie Ende 52 kann dabei wie dargestellt geringfügig außerhalb des Längsseitenrands 46 oder (nicht dargestellt) leicht innerhalb des Längsseitenrands 46 angeordnet sein.

Der andere Abschnitt 42 des Hüftgürtels 4 ist ebenfalls um eine erste Faltachse 54 und um eine zweite Faltachse 56 gefaltet. Hierbei wird die Lage der zweiten Faltachse 56 in Querrichtung 50 vorzugsweise wieder so gewählt, dass das freie Ende 58 des zweiten Abschnitts 42 wie vorausgehend im Zusammenhang mit dem freien Ende 52 des ersten Abschnitts 40 beschrieben gut greifbar im Bereich des anderen Längsseitenrands 60 zu liegen kommt. Die gefalteten Abschnitte 40, 42 überlappen einander in einem Abschnitt 61.

Des Weiteren ist in Figur 6 ein Topsheet 62 und ein Backsheet 64 dargestellt. Man erkennt also, dass die Faltung des Hüftgürtels auf der Außenseite der Wegwerfwindel ausgebildet ist.

Figur 7 zeigt eine weitere Ausführungsform einer erfindungsgemäßen Wegwerfwindel, wonach die zweiten Verschlussmittel 26 als ein einziges in Umfangsrichtung erstrecktes vorzugsweise elastisch dehnbares Band ausgebildet sind. Dieses Band lässt sich, wie aus Figur 7 ersichtlich, in Richtung auf den Schrittbereich der Wegwerfwindel 2 verlagern und dort eine ungefähre V-Form bildend lösbar festlegen.

Eine noch weitere Ausführungsform zeigt Figur 8. Danach sind die zweiten Verschlussmittel 26 insbesondere an beiden Längsenden 28 und 36 unlösbar am Windelhauptteil bzw. am Hüftgürtel 4 festgelegt. Sie weisen aber ungefähr in ihrer Mitte eine lösbare Verbindung 66 auf. Dort sind die Verschlussmittel 26 geteilt oder getrennt, und über wiederum an sich beliebige Verschlusselemente, möglicherweise auch über Schnallen, Haken oder Ösen, lösbar miteinander verbindbar.

## Patentansprüche

1. Wegwerfwindel (2), insbesondere zur Inkontinentenversorgung, mit einem eine in Umfangsrichtung geschlossene Hüftöffnung der Windel bildenden Hüftgürtel (4), der an wenigstens einer Stelle öffenbar und schließbar ist, und mit einem einen Vorderbereich (8), einen Rückenbereich (12) und einen dazwischenliegenden Schrittbereich (10) aufweisenden Windelhauptteil (6), der eine körperabgewandte flüssigkeitsundurchlässige Lage (14) (Backsheet), eine körperzugewandte flüssigkeitsdurchlässige Lage (Topsheet) und einen Absorptionskörper für Körperflüssigkeiten aufweist, wobei der Hüftgürtel (4) unlösbar an den Rückenbereich (12) des Windelhauptteils (6) angefügt ist und der Windelhauptteil (6) mit dem Längsende (18) seines Vorderbereichs (8) über erste Verschlussmittel (20) lösbar am Hüftgürtel (4) festlegbar ist, derart, dass ein Benutzer bei angelegtem Hüftgürtel (4) den Windelhauptteil (6) zwischen den Beinen hervorholen und das noch freie Längsende des Windelhauptteils (6) am Hüftgürtel (4) lösbar festlegen kann, **dadurch gekennzeichnet, dass** zusätzlich zu den zwischen Hüftgürtel (4) und Längsende (18) des Windelhauptteils (6) wirkenden ersten Verschlussmitteln (20) langgestreckt ausgebildete zweite Verschlussmittel (26) mit einer Länge von 10 - 50 cm vorgesehen sind, die eineHalteverbindung zwischen dem Hüftgürtel (4) einenends und dem Vorderbereich (8) oder Schrittbereich (10) des Windelhauptteils (6) anderenends ausbilden können und die elastisch dehnbar und derart anordenbar sind, dass ein Benutzer nach dem Lösen der ersten Verschlussmittel (20) den Vorderbereich (8) des Windelhauptteils (6) zum Wasserlassen vom Hüftgürtel (4) weg nach unten klappen oder falten kann.

2. Wegwerfwindel nach Anspruch 1, **dadurch gekennzeichnet, dass** die zweiten Verschlussmittel (26) eine Länge von 10 - 40 cm, insbesondere 15 - 35 cm aufweisen.

3. Wegwerfwindel nach Anspruch 1 oder 2, **dadurch gekennzeichnet, dass** die zweiten Verschlussmittel (26) eine Breite quer zu ihrer Längserstreckung von 0,5 - 8 cm, insbesondere von 0,5 - 6, insbesondere von 0,5 - 5 cm, insbesondere von 0,5 - 4 cm, insbesondere von 0,5 - 3 cm aufweisen.

4. Wegwerfwindel nach Anspruch 1, 2 oder 3, **dadurch gekennzeichnet, dass** die zweiten Verschlussmittel (26) im angelegten Zustand der Windel etwa V-förmig zueinander verlaufend angeordnet sind.

5. Wegwerfwindel nach einem oder mehreren der vorstehenden Ansprüche, **dadurch gekennzeichnet, dass** die zweiten Verschlussmittel (26) einenends mit dem Hüftgürtel (4) beim bestimmungsgemäßen Gebrauch unlösbar verbunden sind und anderenends mit dem Windelhauptteil lösbar verbindbar sind.

6. Wegwerfwindel nach einem oder mehreren der vorstehenden Ansprüche, **dadurch gekennzeichnet, dass** die zweiten Verschlussmittel (26) einenends mit dem Windelhauptteil (6) beim bestimmungsgemäßen Gebrauch unlösbar verbunden sind und anderenends mit dem Hüftgürtel (4) lösbar verbindbar sind.

7. Wegwerfwindel nach einem oder mehreren der vorstehenden Ansprüche, **dadurch gekennzeichnet, dass** die zweiten Verschlussmittel (26) mit ihren Enden, insbesondere unlösbar am Hüftgürtel (4) und am Windelhauptteil (6) festgelegt sind und entlang ihrer Erstreckung eine lösbar schließbare Verbindung aufweisen.

8. Wegwerfwindel nach einem oder mehreren der vorstehenden Ansprüche, **dadurch gekennzeichnet, dass** die ersten und/oder die zweiten Verschlussmittel (20 oder 26) mechanisch wirkende Verschlusselemente oder klebend wirkende Bereiche aufweisen.

9. Wegwerfwindel nach einem oder mehreren der vorstehenden Ansprüche, **dadurch gekennzeichnet, dass** die zweiten Verschlussmittel (26) über vorpositionierte Gegenverschlusselemente oder -bereiche am Windelhauptteil (6) oder am Hüftgürtel (4) geschlossen werden können.

10. Wegwerfwindel nach einem oder mehreren der vorstehenden Ansprüche, **dadurch gekennzeichnet, dass** die zweiten Verschlussmittel (26) bei der Abgabe an den Verbraucher, insbesondere im verpackten Zustand vor der Ingebrauchnahme leporelloförmig gefaltet.

11. Wegwerfwindel nach einem oder mehreren der vorstehenden Ansprüche, **dadurch gekennzeichnet, dass** die zweiten Verschlussmittel (26) bei der Abgabe an den Verbraucher, insbesondere im verpackten Zustand vor der Ingebrauchnahme entlang dem Hüftgürtel (4) angeordnet oder erstreckt sind.

12. Wegwerfwindel nach einem oder mehreren der vorstehenden Ansprüche, **dadurch gekennzeichnet, dass** die zweiten Verschlussmittel (26) bei der Abgabe an den Verbraucher, insbesondere im verpackten Zustand vor der Ingebrauchnahme, an der flüssigkeitsundurchlässigen Lage angeordnet sind.

13. Wegwerfwindel nach einem oder mehreren der vorstehenden Ansprüche, **dadurch gekennzeichnet, dass** die zweiten Verschlussmittel (26) bei der Abgabe an den Verbraucher, insbesondere im verpackten Zustand vor der Ingebrauchnahme dem Hüftgürtel (4) oder dem Windelhauptteil (6) derart beigeordnet sind, dass sie in dieser Konfiguration auch ungebraucht belassen werden können, ohne störend zu wirken.

14. Wegwerfwindel nach einem oder mehreren der vorstehenden Ansprüche, **dadurch gekennzeichnet, dass** die zweiten Verschlussmittel (26) bei der Abgabe an den Verbraucher, insbesondere im verpackten Zustand vor der Ingebrauchnahme kaschiert sind.

15. Wegwerfwindel nach einem oder mehreren der vorstehenden Ansprüche, **dadurch gekennzeichnet, dass** der Hüftgürtel (4) unlösbar an die flüssigkeitsundurchlässige Lage des Windelhauptteils (6) angefügt ist.

16. Wegwerfwindel nach einem oder mehreren der vorstehenden Ansprüche, **dadurch gekennzeichnet, dass** der Hüftgürtel (4) in Umfangsrichtung einstückig durchgehend ausgebildet ist.

## Claims

1. Disposal diaper (2) in particular for incontinent care, with a hip belt (4) forming a peripherally closed hip opening of the diaper which can be opened and closed at a least one location, and with a main diaper portion (6) having a front region (8), a rear region (12) and an intermediate crotch region (10) as well as a liquid-impermeable layer (14) (back sheet), facing away from a body, liquid-permeable layer (top sheet) facing the body and an absorption body for body fluids, wherein the hip belt (4) is non detachably joined to the rear region (12) of the main diaper portion (6) and the main diaper portion (6) can be attached to the hip belt (4) in a detachable fashion with the longitudinal end (18) of its front region (8) using first closing means (20) in such a fashion that, with the hip belt (4) applied, a user can raise the main diaper portion (6) between the legs and attach the free longitudinal end of the main diaper portion (6) to the hip belt (4) in a detachable fashion, **characterized in that**, in addition to the first closing means (20), which is disposed between the hip belt (4) and the longitudinal end (18) of the main diaper portion (6), longitudinally extended second closing means (26) having a length of 10 to 50 cm are also provided for which can effect a first holding connection between the hip belt (4), at one end, and the front region (8) or the crotch region (10) of the main diaper portion (6), at the other end, and which are elastically stretchable and disposed in such a fashion that a user, after loosening the first closing means (20), can fold or open the front region (8) of the main diaper portion (6) away downwardly from the hip belt (4) for urinating outwardly.

2. Disposable diaper according to claim 1, **characterized in that** the second closing means (26) have a length 10 to 40 cm, and in particular 15 to 35 cm.

3. Disposable diaper according to claim 1 or 2, **characterized in that** the second closing means (26) have a width transverse to their longitudinal extent of between 0.5 and 8 cm, in particular between 0.5 and 6 cm, in particular between 0.5 and 5 cm, in particular between 0.5 and 4 cm, and in particular between 0.5 and 3 cm.

4. Disposable diaper according to claim 1, 2 or 3, **characterized in that** the second closing means (26) extend approximately in a V-shaped manner with respect to each other in the applied state of the diaper.

5. Disposable diaper according to any one or more of the preceding claims, **characterized in that** the second closing means (26) are permanently connected at one side to the hip belt (4) during proper use and are detachably connected to the main diaper portion at the other end thereof.

6. Disposable diaper according to any one or more of the preceding claims, **characterized in that** the second closing means (26) are permanently connected at one end to the main diaper portion (6) during proper use and are detachably connected to the hip belt (4) on the other end thereof.

7. Disposable diaper according to any one or more of the preceding claims, **characterized in that** the second closing means (26) have ends which are attached, in particular in a permanent manner, to the hip belt (4) and to the main diaper portion (6) and have a closeable detachable connection along their longitudinal extent.

8. Disposable diaper according to any one or more of the preceding claims, **characterized in that** the first and/or the second closing means (20 or 26) have mechanically acting closing elements or adhesive acting regions.

9. Disposable diaper according to any one or more of the preceding claims, **characterized in that** the second closing means (26) can be closed by means of prepositioned, opposing closing elements or regions on the main diaper portion (6) or on the hip belt (4).

10. Disposable diaper according to any one or more of the preceding claims, **characterized in that** the second closing means (26), when presented to the user in particular in a packaged state prior to use, are folded in a pleated manner.

11. Disposable diaper according to any one or more of the preceding claims, **characterized in that** the second closing means (26), when presented to the user in particular in a packaged state prior to use, are disposed or extended along the hip belt (4).

12. Disposable diaper according to any one or more of the preceding claims, **characterized in that** the second closing means (26), when presented to the user in particular in a packaged state prior to use, are disposed on the liquid impermeable layer.

13. Disposable diaper according to any one or more of the preceding claims, **characterized in that** the second closing means (26), when presented to the user in particular in a packaged state and prior to use, are associated with the hip belt (4) or the main diaper portion (6) in such a fashion that they can be left unused in this state without interference.

14. Disposable diaper according to one or more of the preceding claims, **characterized in that** the second closing means (26), when presented to the user in particular in a package state prior to use, are covered.

15. Disposable diaper according to any one or more of the preceding claims, **characterized in that** the hip belt (4) is attached in a permanent fashion to the liquid-impermeable layer of the main diaper portion (6).

16. Disposable diaper according to any one or more of the preceding claims, **characterized in that** the hip belt (4) is configured in the peripheral direction as one continuous piece.

## Revendications

1. Couche jetable (2), en particulier pour les soins aux incontinents, comportant une ceinture de taille (4) formant une ouverture de taille de la couche, ouverture fermée dans le sens périphérique, laquelle ceinture de taille peut être ouverte et fermée à au moins un endroit, et comportant une partie principale de couche (6) présentant une zone avant (8), une zone arrière (12) et une zone d'entrejambe (10) située entre celles-ci, laquelle partie principale de couche présente une couche (14) imperméable aux liquides orientée à l'opposé du corps (backsheet), une couche perméable aux liquides orientée vers le corps (topsheet) et un corps d'absorption pour les liquides corporels, la ceinture de taille (4) étant raccordée de manière inamovible à la zone arrière (12) de la partie principale de couche (6) et la partie principale de couche (6) pouvant être fixée de manière amovible à la ceinture de taille (4) avec l'extrémité longitudinale (18) de sa zone avant (8) par le biais des premiers moyens de fermeture (20), de telle sorte qu'un utilisateur puisse, lorsque la ceinture de taille (4) est mise en place, amener vers l'avant la partie principale de couche (6) entre les jambes et fixer de manière amovible l'extrémité longitudinale encore libre de la partie principale de couche (6) sur la ceinture de taille (4), **caractérisée en ce que**, en plus des premiers moyens de fermeture (20) agissant entre la ceinture de taille (4) et l'extrémité longitudinale (18) de la partie principale de couche (6), il est prévu des seconds moyens de fermeture (26) réalisés très allongés d'une longueur comprise entre 10 et 50 cm, lesquels peuvent former une liaison de retenue entre la ceinture de taille (4) d'une part et la zone avant (8) ou la zone d'entrejambe (10) de la partie principale de couche (6) d'autre part et lesquels sont élastiquement extensibles et peuvent être agencés de telle façon que, pour uriner, un utilisateur puisse, après avoir détaché les premiers moyens de fermeture (20), détacher la zone avant (8) de la partie principale de couche (6) de la ceinture de taille (4) en la rabattant ou la pliant vers le bas.

2. Couche jetable selon la revendication 1, **caractérisée en ce que** les seconds moyens de fermeture (26) présentent une longueur de 10 à 40 cm, en particulier de 15 à 35 cm.

3. Couche jetable selon la revendication 1 ou 2, **caractérisée en ce que** les seconds moyens de fermeture (26) présentent, transversalement à leur dimension longitudinale, une largeur de 0,5 à 8 cm, en particulier de 0,5 à 6 cm, en particulier de 0,5 à 5 cm, en particulier de 0,5 à 4 cm, en particulier de 0,5 à 3 cm.

4. Couche jetable selon la revendication 1, 2 ou 3, **caractérisée en ce que** les seconds moyens de fermeture (26), lorsque la couche est mise en place, sont agencés de manière à s'étendre approximativement en forme de V l'un par rapport à l'autre.

5. Couche jetable selon l'une ou plusieurs des revendications précédentes, **caractérisée en ce que** les seconds moyens de fermeture (26) d'une part sont reliés de manière inamovible à la ceinture de taille (4) en cas d'utilisation conforme à l'usage défini et d'autre part peuvent être reliés de manière amovible à la partie principale de couche.

6. Couche jetable selon l'une ou plusieurs des revendications précédentes, **caractérisée en ce que** les seconds moyens de fermeture (26) d'une part sont reliés de manière inamovible à la partie principale de couche (6) en cas d'utilisation conforme à l'usage défini et d'autre part peuvent être reliés de manière amovible à la ceinture de taille (4).

7. Couche jetable selon l'une ou plusieurs des revendications précédentes, **caractérisée en ce que** les seconds moyens de fermeture (26) sont fixés avec leurs extrémités, notamment de manière inamovible, sur la ceinture de taille (4) et sur la partie principale de couche (6) et présentent le long de leur étendue une liaison pouvant être fermée de façon détachable.

8. Couche jetable selon l'une ou plusieurs des revendications précédentes, **caractérisée en ce que** les premiers et/ou les seconds moyens de fermeture (20 ou 26) présentent des zones agissant de façon adhésive ou des éléments de fermeture agissant de façon mécanique.

9. Couche jetable selon l'une ou plusieurs des revendications précédentes, **caractérisée en ce que** les seconds moyens de fermeture (26) peuvent être fermés par le biais de contre-éléments ou zones de fermeture prépositionné(e)s sur la partie principale de couche (6) ou sur la ceinture de taille (4).

10. Couche jetable selon l'une ou plusieurs des revendications précédentes, **caractérisée en ce que** les seconds moyens de fermeture (26) sont pliés en accordéon lors de la remise à l'utilisateur, en particulier dans l'état emballé avant l'utilisation.

11. Couche jetable selon l'une ou plusieurs des revendications précédentes, **caractérisée en ce que** les seconds moyens de fermeture (26) s'étendent ou sont agencés le long de la ceinture de taille (4) lors de la remise à l'utilisateur, en particulier dans l'état emballé avant l'utilisation.

12. Couche jetable selon l'une ou plusieurs des revendications précédentes, **caractérisée en ce que** les seconds moyens de fermeture (26) sont agencés sur la couche imperméable aux liquides lors de la remise à l'utilisateur, en particulier dans l'état emballé avant l'utilisation.

13. Couche jetable selon l'une ou plusieurs des revendications précédentes, **caractérisée en ce que** les seconds moyens de fermeture (26) sont adjoints à la ceinture de taille (4) ou à la partie principale de couche (6) lors de la remise à l'utilisateur, en particulier dans l'état emballé avant l'utilisation, de telle façon que, dans cette configuration, ils puissent aussi être laissés inutilisés sans être gênants.

14. Couche jetable selon l'une ou plusieurs des revendications précédentes, **caractérisée en ce que** les seconds moyens de fermeture (26) sont contrecollés lors de la remise à l'utilisateur, en particulier dans l'état emballé avant l'utilisation.

15. Couche jetable selon l'une ou plusieurs des revendications précédentes, **caractérisée en ce que** la ceinture de taille (4) est raccordée de manière inamovible à la couche imperméable aux liquides de la partie principale de couche (6).

16. Couche jetable selon l'une ou plusieurs des revendications précédentes, **caractérisée en ce que** la ceinture de taille (4) est réalisée d'un seul tenant de bout en bout dans le sens périphérique.
